# FASCICULE DE BREVET EUROPEEN

(11) **EP 0 467 980 B1**
(45) Date de publication et mention de la délivrance du brevet: **03.03.1993**
(21) Numéro de dépôt: 90907097.1
(22) Date de dépôt: 04.04.1990
(51) Int. Cl.: G01H 1/00

(54) **PROCEDE ET DISPOSITIF DE MESURE DE VIBRATIONS, ET EN PARTICULIER DU TREMBLEMENT NERVEUX DES ORGANISMES VIVANTS**
VERFAHREN UND ANORDNUNG FÜR DAS MESSEN VON VIBRATIONEN, INSBESONDERE DES NERVÖSEN TREMORS VON LEBENDEN ORGANISMEN
PROCESS AND DEVICE FOR MEASURING VIBRATIONS, IN PARTICULAR NERVOUS TREMBLING IN LIVING ORGANISMS

(30) Priorité: 10.04.1989 FR 8904674
(43) Date de publication de la demande: 29.01.1992
(73) Titulaire: COMBY, Bruno, Soisy-Bouy, F-77650 Longueville (FR); BURGER, Guy-Claude, Soisy-Bouy, F-77650 Longueville (FR)
(72) Inventeur: COMBY, Bruno, Soisy-Bouy, F-77650 Longueville (FR); BURGER, Guy-Claude, Soisy-Bouy, F-77650 Longueville (FR)
(74) Mandataire: CABINET BONNET-THIRION
(86) Numéro de dépôt international: FR9000232
(87) Numéro de publication internationale: WO9012293

(56) Documents cités:
- EP-A- 87 015
- FR-A- 2 577 791

## Description

La présente invention concerne un procédé et un dispositif selon ce procédé permettant de mesurer des vibrations.

Les vibrations mesurées peuvent être celles d'un objet, d'un animal ou d'un homme.

De nombreux appareils permettent la mesure de vibrations. L'invention est notamment caractérisée par le fait que l'on mesure les vibrations d'un objet en distinguant les phases de tremblement de faible amplitude de l'objet des phases de tremblement plus importantes dues à des stimulations parasites, de manière à ne prendre en compte que les valeurs les plus basses (tremblement au repos) tout en éliminant les plus hautes. Une manière de distinguer les phases de tremblement au repos des phases de tremblement d'amplitude plus élevée dues à des stimulations parasites consiste à mesurer le minimum absolu de l'amplitude des vibrations d'un objet pendant une durée prédéterminée. Ainsi, une vibration brusque et inattendue pendant la mesure (sursaut, choc...) n'influe pas sur le résultat.

Les applications de l'invention aux mesures du tremblement nerveux des organismes vivants sont particulièrement intéressantes car l'amplitude du tremblement au repos d'un animal ou d'un homme augmente lorsque celui-ci est énervé, fatigué ou qu'il a absorbé des substances non-naturelles (tabac, café, aliments artificiels...). Cependant la simple mesure de l'amplitude du tremblement ne suffit pas car le signal généré par le capteur résulte aussi bien des tremblements microscopiques (qui nous intéressent) que des mouvements musculaires macroscopiques dont l'amplitude est bien plus importante. Un filtrage sélectif de certaines fréquences pour distinguer le tremblement au repos du tremblement en action (ou tremblement musculaire) n'est pas possible car le tremblement au repos et le tremblement musculaire sont sur les mêmes fréquences, de l'ordre de 10 Hz chez l'homme. Le procédé et le dispositif selon l'invention présentent l'avantage, en distinguant bien le tremblement au repos par rapport aux phases de tremblement plus importantes dues à des vibrations parasites, de faire ressortir seulement le tremblement au repos, les mouvements de l'animal pendant la durée de la mesure n'influant pas sur le résultat.

La mesure du stress chez l'homme est traditionnellement effectuée soit par la mesure de la température corporelle, soit par la mesure du rythme cardiaque. Mais ces paramètres ne donnent qu'une indication approximative du degré de nervosité et, de plus, les dispositifs proposés au public, en particulier ceux qui mesurent la température des doigts à l'aide de substances sensibles à la température, sont très imprécis.
Le dispositif selon l'invention quantifie le stress de manière objective et avec précision à partir d'une autre grandeur physiologique que celles précédemment utilisées : il s'agit de quantifier le tremblement de l'organisme au repos, tremblement qui est invisible ou à peine visible à l'oeil nu chez l'homme. La mesure des vibrations de l'organisme humain comme indicateur de l'état de stress est nouvelle à la connaissance des demandeurs. La présente invention offre l'avantage de donner un résultat plus précis que les précédents dispositifs et qui est plus en rapport avec le degré de nervosité réel. La possibilité de mesurer le stress par la présente invention résulte du fait qu'une réaction de stress s'accompagne d'une décharge d'adrénaline qui augmente l'amplitude du tremblement nerveux.

Les expériences ont montré que le résultat des mesures de vibrations effectuées selon le dispositif de la présente invention augmente effectivement chez les individus en situation de stress et chez ceux qui fument du tabac ou qui ingèrent des aliments non-naturels, le niveau de tremblement étant alors de 3 à 5 fois plus élevé que dans des conditions normales. Au contraire, le résultat des mesures diminue en quelques jours chez les individus qui arrêtent de fumer, de boire du café et/ou qui adoptent une alimentation plus naturelle. Le tremblement diminue également entre le début et la fin d'une séance de relaxation, ce qui d'une part confirme que la grandeur mesurée est un bon reflet de l'état de nervosité, et d'autre part débouche sur la possibilité d'utiliser l'invention pour évaluer l'efficacité de méthodes anti-stress (relaxation, thalassothérapie...). On ne connait actuellement aucun moyen fiable de mesure du stress et par conséquent aucun moyen permettant de mesurer l'efficacité de ces méthodes ou de les comparer entre elles, ce que permet l'invention. Les expériences des inventeurs ont également montré que la moindre introduction de substance non-naturelle dans un organisme vivant (nicotine, caféine, aliments dénaturés par la cuisson ou par irradiation, pain...), même en faible quantité, augmente presque immédiatement les vibrations telles qu'elles sont mesurées par le dispositif décrit, ce qui permet de détecter même une toute petite proportion de substances anormales dans les aliments.

On connaît, par le brevet FR 2 577 791, un procédé et un dispositif pour l'évaluation des effets psychotropes de substances médicamenteuses chez les animaux par la mesure de l'agitation significative de ceux-ci. Le traitement du signal ainsi que les capteurs de la présente invention sont cependant très différents de ceux précédemment décrits dans ce brevet français. En particulier, le principe de la mesure du tremblement au repos (traitement du signal qui ne prend en compte que les signaux de faible amplitude, par exemple par la détection d'un minimum, tout en ne tenant pas compte des signaux parasites d'amplitude plus élevée) est inédit, les procédés antérieurs mesurant l'ensemble de l'agitation significative d'un animal et non l'activité au repos.

Le brevet EU 0 087 015 décrit un dispositif destiné à alerter un éleveur qu'une vache ou des animaux d'élevage vont mettre bas. Le dispositif décrit détecte les contractions de l'utérus de l'animal au moyen d'un capteur à mercure qui se ferme au dessus d'un certain seuil de contractions. Ce procédé est totalement différent de la présente invention par le fait que la présente invention mesure l'amplitude d'un tremblement au repos, tandis que le brevet EU 0 087015 mesure au contraire les contractions de nature musculaire au dessus d'un certain seuil de mouvement. Le traitement du signal diffère donc totalement : dans la présente invention, on ne mesure pas une activité musculaire au dessus d'un certain seuil, mais l'amplitude du tremblement au repos, en éliminant au contraire le signal d'origine musculaire considéré comme un signal parasite. De plus, le capteur du brevet 0 087 015 fournit un signal binaire (ouvert/fermé) qui est évidemment traité très différemment du signal de la présente invention, dont le capteur piézo-électrique fournit un signal analogique.

Les caractéristiques et les avantages de l'invention ressortiront d'ailleurs de la description qui va suivre à titre d'exemple non limitatif, en référence aux dessins annexés dans lesquels:
- Les figures 1 et 2 sont des illustrations schématiques de deux formes de réalisation.
- La figure 3 montre un exemple de capteur pour les mesures chez l'animal.
- La figure 4 montre un exemple de capteur piézo-électrique bien adapté aux mesures chez l'homme.
- La figure 5 est un schéma-bloc.
- Les figures 6A à 6G présentent un schéma électronique de réalisation.
- La figure 7 montre une forme de réalisation avec capteur intégré dans le boitier.
- La figure 8 montre la courbe de réponse du capteur de la figure 4.

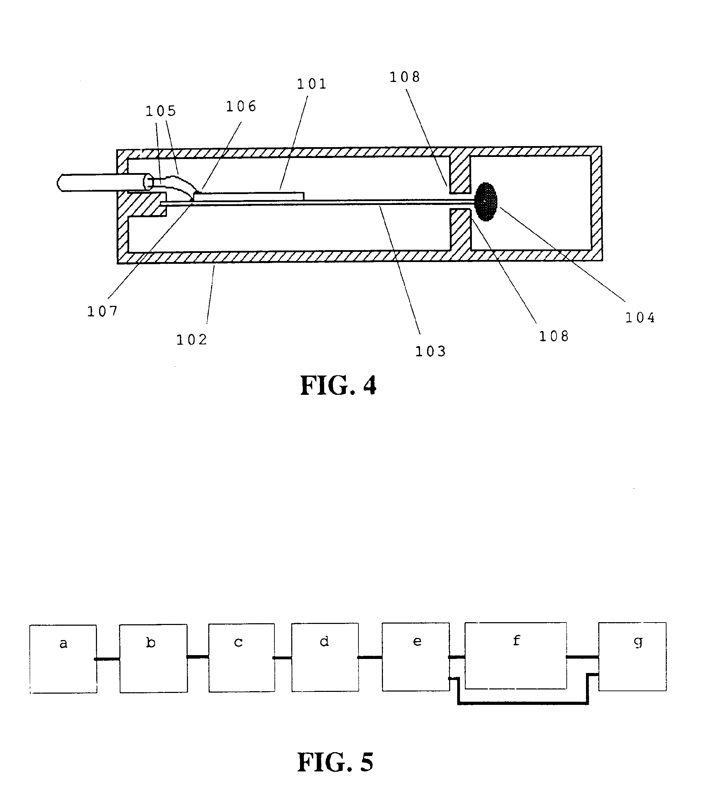

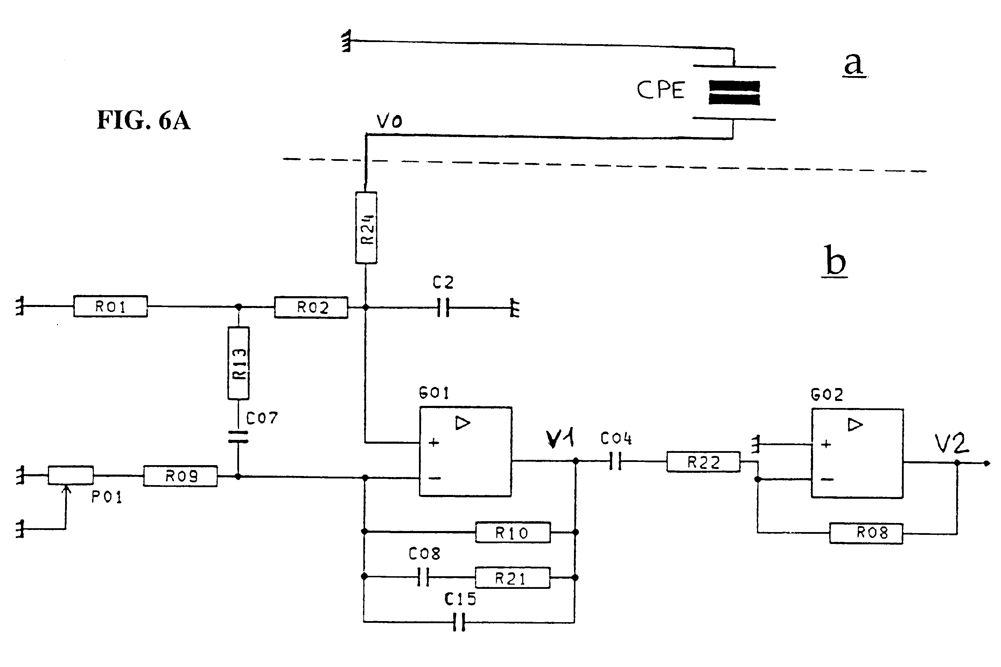

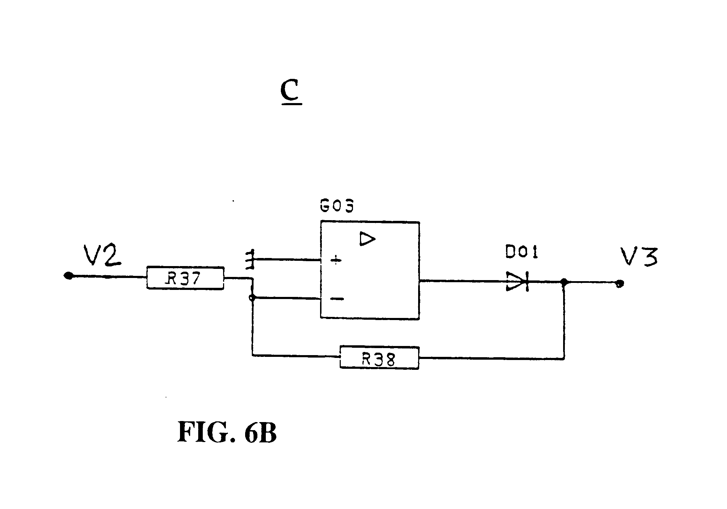

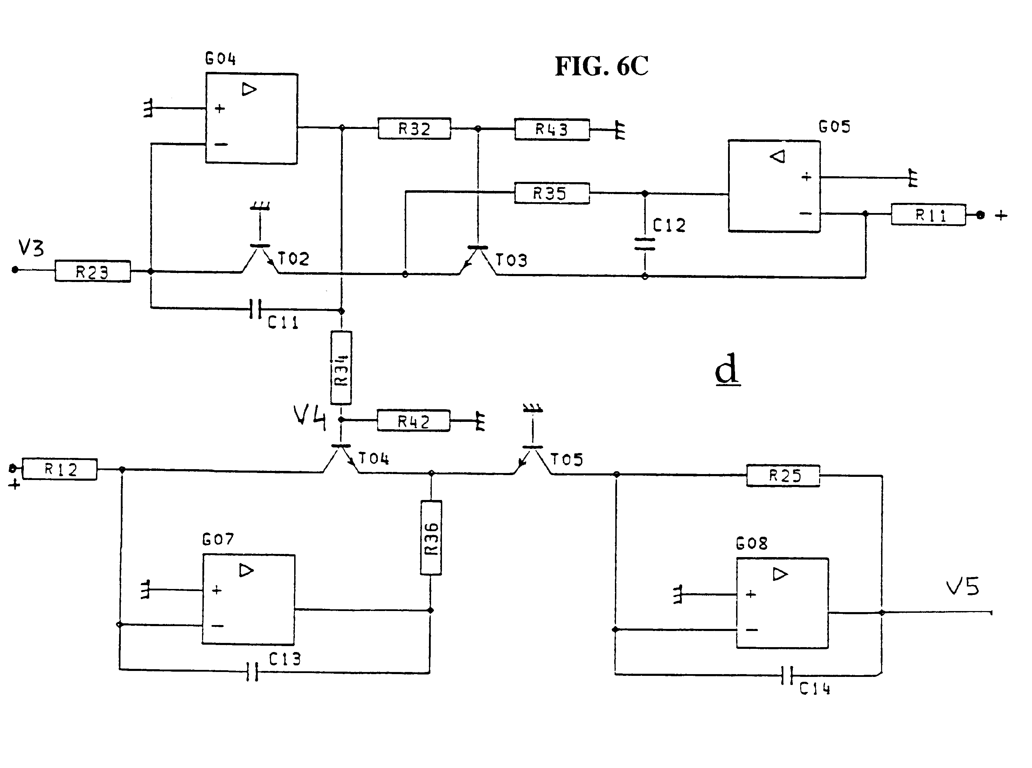

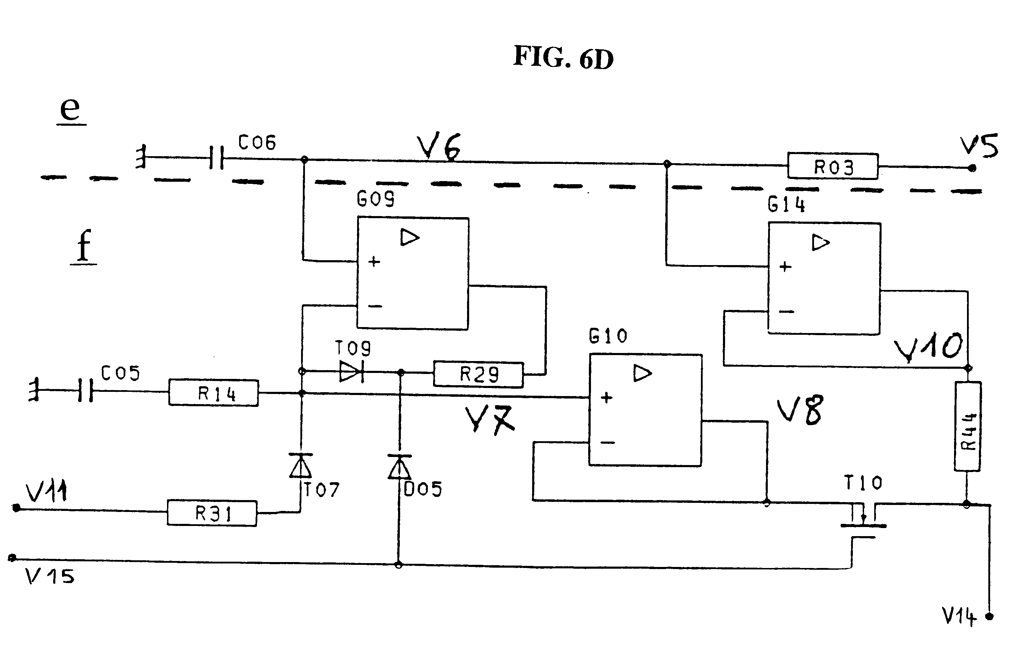

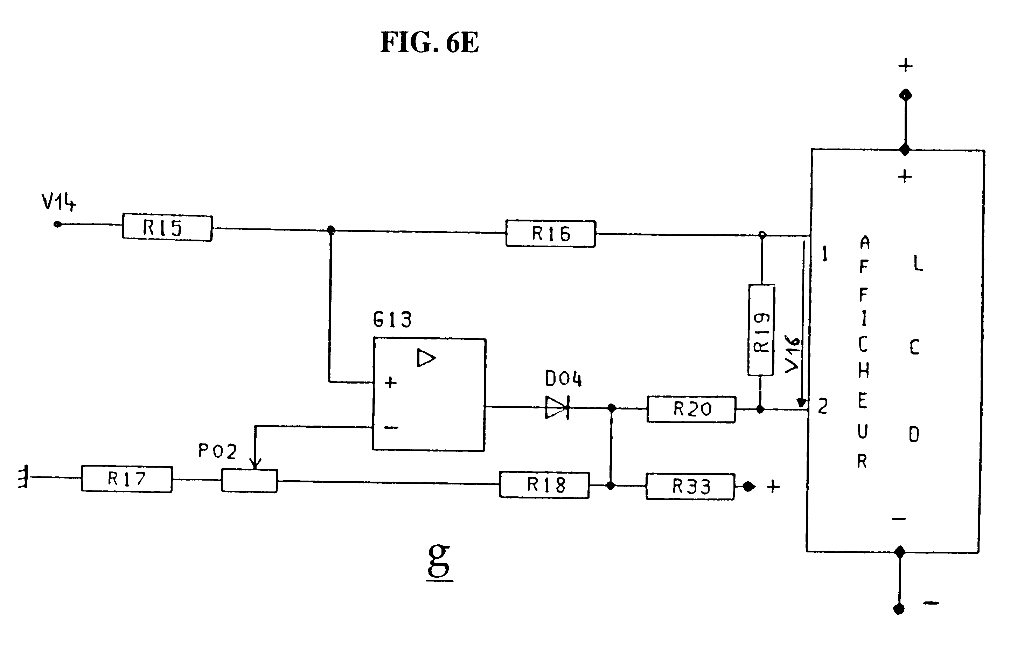

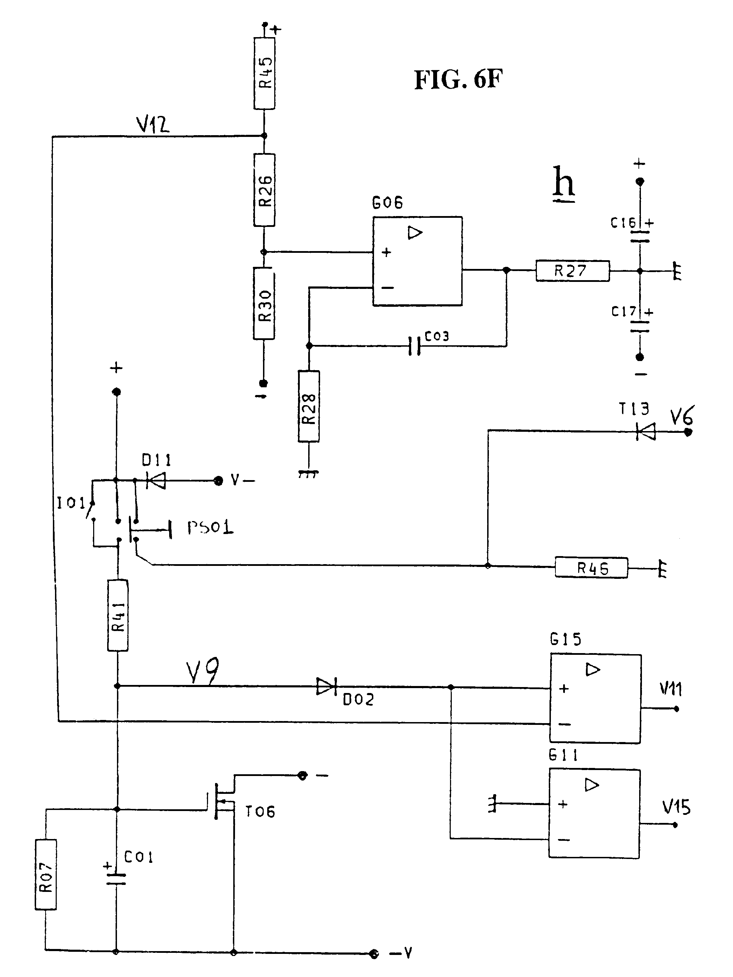

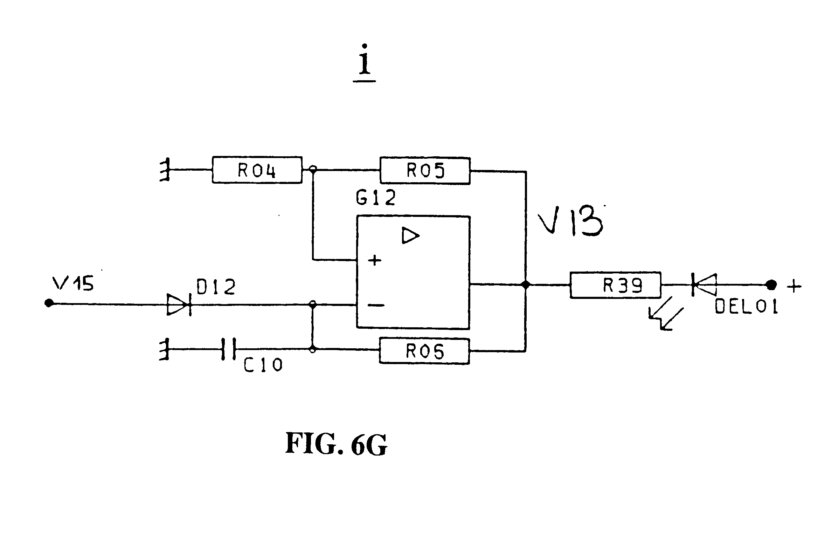

La mesure de vibrations peut comporter notamment les opérations suivantes (les numéros renvoient aux blocs des figures 5 et 6) :
transformation des vibrations en signal électrique, au moyen d'un capteur piézo-électrique (0) --- amplification par un circuit amplificateur (1) --- filtrage par des filtres passe-haut et passe-bas --- redressement par circuit redresseur double alternance (2) --- élévation au carré par un circuit quadrateur (3) --- lissage du signal par un filtre passe-bas) (4) --- détection d'un minimum (5) --- affichage ou transmission du résultat vers d'autres appareils (6).

Les deux premiers filtres passe-haut et passe-bas permettent de sélectionner une gamme de fréquences. Par exemple, pour la mesure du tremblement humain, la gamme des fréquences intéressantes est de 3 à 20 Hz environ. En filtrant les fréquences inférieures à 3 Hz et supérieures à 20 Hz, on évite d'éventuels signaux parasites provenant par exemple de résonnances mécaniques (fréquences basses) ou d'induction par le secteur (50 Hz).

Le circuit électronique de traitement du signal délivré par le capteur pourra donc comporter un circuit d'amplification (1), un redresseur (2), des filtres et notamment un filtre passe-bas pour le lissage (4), et la détection d'un minimum (5). On pourra éventuellement y rajouter un circuit correcteur en fonction de la fréquence et une élévation au carré (quadrateur)(3).

Différentes variantes peuvent être envisagées quant à la façon d'éliminer les signaux parasites correspondant à une amplitude élevée pour ne garder que le tremblement au repos. Par exemple, on peut mesurer le mininum absolu du signal pendant la mesure. Il est aussi possible de mesurer la moyenne des minima atteints pendant la durée prédéterminée de la mesure ou le nombre de passages en dessous d'un seuil prédéterminé de tremblement, ou le temps pendant lequel le signal est resté inférieur à un certain seuil, etc. Le point commun entre ces différents procédés réside dans le fait qu'on ne s'intéresse qu'aux valeurs les plus faibles de l'amplitude des vibrations, en éliminant les valeurs les plus élevées considérées comme un signal parasite sans intérêt. Un tel traitement du signal peut évidemment être combiné avec un filtrage sélectif de certaines fréquences des vibrations.

Les différents éléments du dispositif selon l'invention : capteur, circuit électronique et affichage peuvent être ou non réunis dans un même boitier.

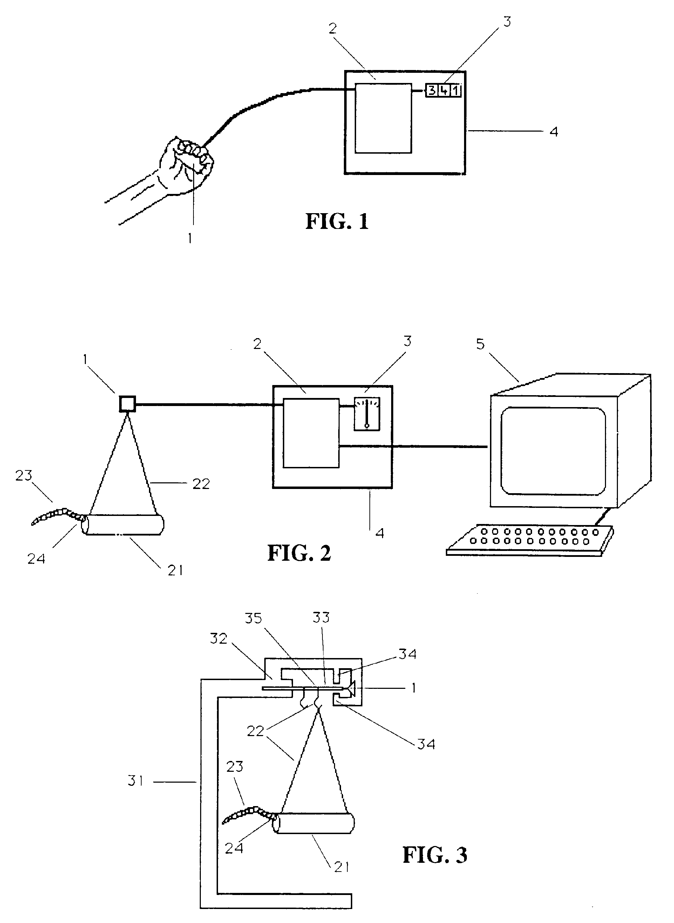

La figure 1 représente un mode de réalisation du dispositif selon l'invention destiné aux applications grand-public (mesure du stress) et médicales. Le capteur (1) peut par exemple être tenu dans la main. Le capteur est relié au boitier de l'appareil par un fil électrique qui sera de préférence blindé (coaxial) et qui achemine le signal électrique vers le circuit (2). Dans cet exemple, le boitier (4) de l'appareil contient à la fois le circuit (2) et l'affichage (3). L'ensemble peut être alimenté sur piles ou sur secteur.

La figure 2 représente une autre réalisation préférentielle plutôt destinée aux applications de laboratoire. Le capteur (1) est par exemple rendu solidaire d'une nacelle (21) contenant une souris ou un autre animal (23) au moyen d'un organe de liaison (22). Les résultats peuvent être transmis à un ordinateur ou système d'enregistrement ou d'impression (5). Le boitier (4) de l'appareil contient à la fois le circuit (2) et l'affichage (3) et est relié d'une part au capteur (1) et d'autre part aux périphériques (5).

La figure 3 montre un modèle de capteur plus particulièrement destiné aux mesures des vibrations des rougeurs qui est constitué d'une cellule piézo-électrique (1) rendue solidaire d'une lame vibrante (33) reliée d'une part à un socle fixe (32), et d'autre part par l'intermédiaire d'un organe de liaison (22) à l'objet de la mesure (23), la course de la lame étant limitée par butée (34) ce qui évite une détérioration de la lame en cas de traction anormalement forte sur l'organe de liaison (22). La position de la jonction entre l'organe de liaison (22) et la lame (33) est réglable par déplacement du point de jonction (35). Ceci présente l'avantage de pouvoir régler la sensibilité du capteur. On n'utilisera par exemple pas la même sensibilité pour mesurer les vibrations d'une souris et celles d'un rat.
L'organe de liaison (22) peut en particulier être une suspension, par exemple au moyen d'un ou de plusieurs fils ou de tout autre système de suspension, le capteur étant situé sur une potence (31).
La longueur du dispositif de suspension (22) est calculée de façon à ce que les oscillations spontanées de la nacelle sous l'effet des mouvements de la souris (mouvements de balançoire) soient filtrées par le circuit et ne faussent pas le résultat de la mesure.
Dans le cas où l'objet de la mesure est un animal, un mode de réalisation du procédé selon l'invention consiste à placer l'animal dans une nacelle (21) de forme et de dimensions telles que l'animal puisse pénétrer dans la nacelle mais qu'il ne puisse pas se retourner à l'intérieur. Ceci présente l'avantage de limiter au maximum les mouvements de l'animal dans la nacelle. Un moyen de visualisation, par exemple un orifice ou une fenêtre (24), permet de détecter instantanément la présence ou non d'un animal (23) dans la nacelle.

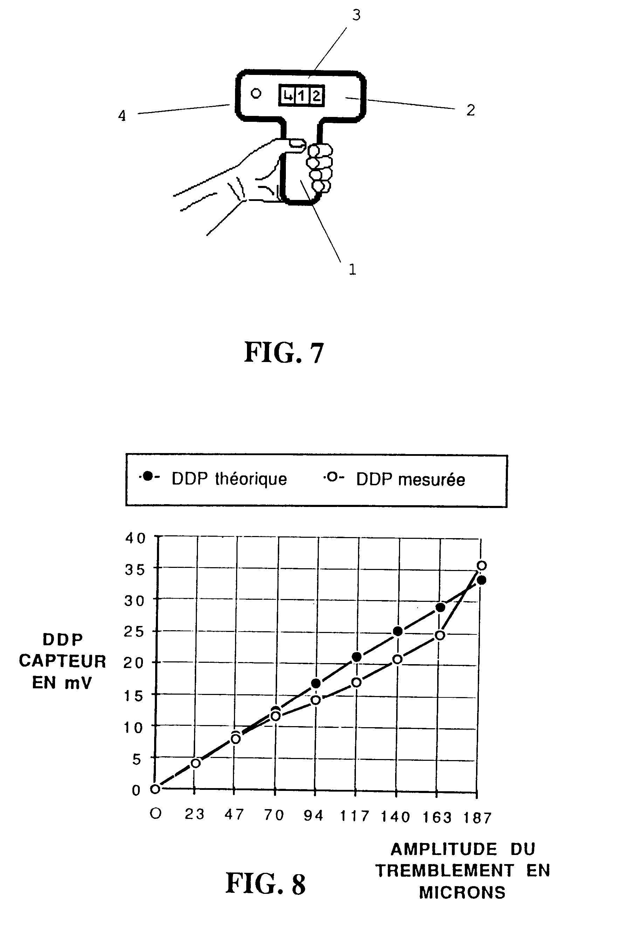

La figure 4 montre une vue en coupe d'un capteur d'accélération facile à industrialiser et de faible coût pour la mise en oeuvre de l'invention. Ce capteur est particulièrement destiné à être tenu dans la main pour les mesures du tremblement humain. Il est réalisé à partir d'un élément piézo-électrique tel qu'une céramique piézo-électrique (101) qui produit entre ses deux faces (106) et (107) une différence de potentiel proportionnelle à sa flexion. L'élément piézo-électrique est rendu solidaire d'un équipage mobile en flexion, par exemple une lame flexible (103). Une extrémité de l'équipage mobile (103) est solidaire du boitier du capteur (102), par exemple par encastrement, collage ou soudure, tandis que l'autre est pourvue d'une masselotte (104). Les mouvements de l'équipage mobile (103) engendrés par l'inertie de la masselotte (104) par rapport aux mouvements du boitier (102) produisent une excitation de l'élément piézo-électrique (101). L'ensemble fonctionne ainsi comme un capteur d'accélérations. La course de l'équipage mobile ou de la masselotte est limitée par des butées (108), ce qui évite d'endommager le capteur en cas de mouvements brusques ou de choc. Un tel capteur fabriqué avec une céramique piézo-électrique collée sur une lame métallique flexible produit ainsi une DDP alternative de même fréquence que la fréquence du tremblement et dont l'amplitude est proportionnelle à l'amplitude du tremblement, ainsi que le montre la courbe de réponse présentée sur la figure 8. Le signal délivré par le capteur est transmis au circuit par deux fils électriques (105) soudés sur chaque face de la céramique (106 et 107). Ces fils (105) sont choisis suffisamment souples pour ne pas entraver par leur rigidité la flexion de la céramique. Pour la même raison, il est avantageux de réaliser les soudures (106 et 107) aussi petites que possible et aux extrémités de la céramique côté boitier plutôt qu'en son centre. Un tel capteur est facile à réaliser de manière industrielle et peu coûteuse et convient particulièrement bien à la mise en oeuvre de l'invention. Une variante de ce capteur peut être réalisée avec une jauge extensométrique fixée sur une lame flexible. La fermeture d'un contact électrique au niveau des butées (108) peut éventuellement permettre d'avertir l'utilisateur par un moyen de visualisation, tel qu'une diode électro-luminescente, que le capteur est en surcharge ou endommagé.
Il est possible de réaliser un autre type de capteur en utilisant une cellule de tourne-disque à laquelle on rajoute une masselotte à l'extrémité du saphir.
Encore un autre modèle de capteur consiste à utiliser à la place d'un capteur d'accélérations un élément piézo-électrique relié à l'objet de la mesure, directement ou par l'intermédiaire d'un ou de plusieurs organes de liaison.
Le capteur destiné à la mesure du tremblement chez l'homme sera préférentiellement en contact avec la main du sujet ou rendu solidaire de n'importe quelle autre partie de l'organisme dont on souhaite mesurer le tremblement.
Le capteur pourra être uni-directionnel ou pluri-directionnel. Cependant, afin de réduire le prix de revient et de faciliter le traitement du signal, il est préférable d'utiliser de préférence un capteur uni-directionnel. Celui-ci, dans le cas de la mesure du tremblement humain, pourra par exemple. être tenu dans la main. Afin de toujours mesurer le même paramètre physiologique, il faut définir la position du sujet et la manière de tenir le capteur pendant la mesure. Les demandeurs ont utilisé la position standard suivante : le sujet debout, pieds à peine écartés, tient le capteur dans sa main droite pour les droitiers, gauche pour les gauchers, en serrant modérement le capteur, le bras à peine fléchi et légérement écarté du corps.
L'utilisation d'un capteur dont la forme n'est pas symétrique, par exemple un capteur avec une indication "dessus" et "dessous" ou un capteur moulé avec la forme des doigts dans la position où il doit être tenu, permet d'imposer l'orientation du capteur et donc d'être certain que le sujet mesurera toujours la même composante du tremblement.
Le réglage de la sensibilité électrique du capteur peut être effectué par un condensateur connecté aux bornes de la céramique ou de la cellule piézo-électrique. En effet pour une force donnée, la quantité d'électricité Q délivrée par la cellule est constante Q=CV donc V=Q/C, c'est à dire que la tension aux bornes du capteur est fonction de C.
Le capteur peut éventuellement être situé dans un petit boitier séparé du reste de l'appareil, le signal étant transmis au circuit électronique :
- soit au moyen d'un fil électrique.
- soit par un dispositif d'émission radio transmettant le signal du capteur doté d'un émetteur vers le circuit doté d'un récepteur.

La figure 5 présente un schéma-bloc des fonctions essentielles réalisées par le circuit des figures 6A à 6G. Le bloc a représente un capteur piézo-électrique. Le bloc breprésente un amplificateur. Le bloc c représente un redresseur. Le bloc d représente un quadrateur. Le bloc e représente un filtre. Le bloc f représente un circuit comportant une mémoire de minimum. Le bloc g représente un affichage.

Les figures 6A à 6G présentent un schéma électronique pour la mise en oeuvre de l'invention dont nous expliquons ci-dessous à titre d'exemple les grandes lignes du fonctionnement :
Les lettres a, b, c, d, e, f, g, sur les figures 6A à 6G renvoient aux références des blocs de la figure 5.

Le bloc a de la figure 6A représente le capteur piézo-électrique CPE qui produit une différence de potentiel alternative V0. Il s'agit en l'occurence d'une céramique piézo-électrique dont l'impédance est équivalente à celle d'un condensateur de 1 nF qui, en parallèle avec C2, et avec l'impédance d'entrée de l'amplificateur G01, constitue un filtre passe-haut avec une fréquence de coupure de l'ordre de 1,5 Hz.

Le bloc b de la figure 6A, l'amplificateur-correcteur, est constitué d'un premier amplificateur G01 de gain réglable par P01. L'impédance d'entrée est très élevée, ceci est dû à la réaction provoquée par C07, relié par R 13 au point commun de R01 et R02. Aux très basses fréquences l'impédance d'entrée est R01+R02. Le réseau constitué de R10 en parallèle avec R21, C08 et C15 constitue un réseau correcteur 1/√F dont l'atténuation en fréquence est de 10 décibels par décade. La valeur de P01 est faible devant celle du réseau correcteur. R10 est beaucoup plus grande que P01 ce qui peut entraîner une tension continue de décalage au point V1. Un deuxième amplificateur G02 relié à V1 par R22 et C04 annule la tension de décalage au point V1. L'entrée positive de l'amplificateur G02 est reliée à la masse, tandis que l'entrée négative est reliée d'une part à R08 dont l'autre extrémité est reliée à la sortie V2 de G02, et d'autre part à V1 par R22 et C04. Le signal V2 est égal au signal V0 multiplié par un coefficient d'amplification et corrigé en 1/√F.

Le bloc c présenté sur la figure 6B est un redresseur double alternance constitué d'une diode D01, d'un ampli redresseur G03 et de deux résistances R37 et R38 de même valeur. Le signal V3 est donc égal à la valeur absolue du signal V2.

Le bloc d présenté sur la figure 6C élève au carré le signal V3. Il s'agit d'un circuit qui comprend 2 parties :
- une première partie qui élabore une tension V4 proportionnelle au logarithme népérien de la tension en V3.
- une deuxième partie qui délivre au point V5 une tension proportionnelle à l'exponentielle de V4.

Par le choix convenable du coefficient multiplicateur donné par le pont diviseur R34-R42, nous obtenons donc V5 =(V3)².

Le bloc e de la figure 6D a pour fonction de lisser le signal redressé (filtrage des hautes fréquences), ce qui est réalisé par le filtre passe-bas R03-C06. La constante de temps est de l'ordre de 1 à 2 secondes. Ce filtrage évite qu'un arrêt ou une diminution du tremblement de courte durée ne soit pris en compte par le circuit de détection du minimum (en particulier le passage à zéro à chaque alternance d'un signal sinusoïdal). L'entrée du filtre est V5 et la sortie V6, point commun de R03 et C06. La deuxième borne de C06 est à la masse tandis que l'autre borne de R03 reçoit le signal V5 à traiter. Le signal lissé V6 est acheminé d'une part vers l'affichage par l'intermédiaire d'un ampli adaptateur d'impédance G14 et d'autre part vers un circuit de détection du minimum.

Le bloc f de la figure 6D effectue la détection du minimum absolu, depuis le début de la mesure, du signal V6, ce minimum étant stocké dans une capacité mémoire C05 et donné par la tension en V7, tandis que la valeur instantanée du tremblement est donnée par la tension en V6 et V10. Le début et la fin de la mesure est déterminé par la tension de commande V11 et la fin de la mesure par la tension de commande V15. La détection du minimum est effectuée par un ampli détecteur de minimum G09, dont la borne positive est reliée à la valeur instantanée du signal V6, l'entrée négative étant reliée via R14 à la capacité mémoire C'05. La sortie de l'ampli détecteur de minimum G09 est reliée à son entrée négative et, à travers R29, à la cathode d'une diode T09, dont l'anode est reliée à C05 à travers R 14, l'autre extrémité de C05 étant à la masse. Le point V7 correspondant à l'entrée négative de l'ampli détecteur de minimum G09, qui contient le résultat de la mesure du minimum, achemine le signal vers l'entrée positive d'un amplificateur adaptateur d'impédance G10 de gain 1 dont l'entrée négative est reliée à sa sortie en V8, le signal V8, égal à V7 mais de faible impédance, étant destiné à l'affichage. L'amplificateur adaptateur d'impédance G14 monté comme G10 est destiné à permettre l'affichage du signal V6, en élaborant la tension V10 égale à V6, mais sous une faible impédance. Un transistor inverseur VMOS T10 relié à V8 d'une part, et à V10 par R44 d'autre part, permet d'obtenir en V14 une tension à afficher égale à V8 ou V10 selon l'état de la tension de commande V15. Le transistor inverseur T10 permet, lorsqu'il est bloqué par une valeur négative de sa commande V15, d'obtenir en V14 la même tension qu'en V10, R44 étant beaucoup plus faible que R15 (voir R15 sur la figure 6E). Lorsque T10 devient conducteur par une valeur positive de sa tension de commande V15, la tension V8 se retrouve en V14.

Le bloc g présenté sur la figure 6E permet l'affichage du résultat de la mesure et/ou de la valeur instantanée du tremblement sur un afficheur digital LCD. Ce bloc comporte en amont de l'afficheur un amplificateur différentiel G13 qui permet de transposer la tension V14 en une tension dont la référence n'est plus la masse, mais l'entrée 1 de l'afficheur dont la tension est imposée. L'entrée négative de l'amplificateur différentiel G13 est reliée au point milieu d'un potentiomètre P02 qui permet de régler le zéro de l'affichage en l'absence de tremblements. La tension affichée V16 est donc égale pendant la mesure à la valeur instantanée du tremblement, et après la mesure au minimum absolu du tremblement pendant la durée de la mesure.

Le bloc h présenté sur la figure 6F est un bloc de commande. Ce bloc comprend notamment un transistor de mise hors-tension T06, un organe de déclenchement PS01, qui est en l'occurence un bouton-poussoir dont t'activation signale le début de la mesure, un commutateur 101 qui permet une autre utilisation du dispositif, une temporisation consistant en une capacité de temporisation C01 se déchargeant dans une résistance de temporisation R07, un ampli de commande G15 qui délivre une tension de commande V11, et un ampli de commande G11 qui délivre une tension de commande V15. La mesure est lancée en activant le bouton-poussoir PS01 qui va d'une part initialiser la temporisation C01-R07, qui détermine la durée de la mesure, en chargeant très rapidement la capacité de temporisation C01 à travers une résistance R41, et d'autre part va initialiser la capacité mémoire C05 à une valeur supérieure au résultat de la mesure attendue par la tension de commande V11 à travers R31 et T07.
La temporisation C01-R07 impose le séquencement suivant :
- La mise sous tension de l'appareil résulte de l'activation de l'organe de déclenchement PS01 qui, en chargeant la capacité de temporisation C01 à un potentiel positif à travers R41, active la grille du transistor VMOS de mise sous tension T06 et le rend conducteur, ce qui alimente la borne (-) du circuit. Simultanément, lors de la pression sur l'organe de déclenchement PS01, une tension de l'ordre de 0,3 Volt est imposée en V6 via T13. L'ampli de commande G15 délivre une tension V11 positive qui impose à travers T07 une tension en V7 égale à V6 égale à 0,3 Volt. Pendant l'activation de l'organe de déclenchement PS01, l'ampli de commande G11 délivre une tension de commande V15 négative qui provoque le clignotement de la diode de visualisation DEL01.
- ensuite, quelques instants après l'activation de l'organe de déclenchement PS01, la tension V9 devient supérieure à la tension de référence V12 déterminée par le pont diviseur R26-R45. L'ampli de commande G15 délivre alors une tension de commande V11 positive ce qui permet d'initialiser au début de la mesure à travers T07 la capacité mémoire C05 à une valeur V7 égale à la valeur instantanée du signal V6. Simultanément la tension de commmande V15 autorise toujours le clignotement de la diode de visualisation DEL01. La tension de commande V15 du transistor inverseur T10 étant toujours positive, la tension affichée depuis l'activation du bouton poussoir et pendant toute la durée de la mesure est égale à la valeur instantanée du tremblement (V14=V10= V6). De même, lorsque le commutateur I01 est en position fermée, la tension affichée est égale à la valeur instantanée du tremblement V6, et ceci pendant une durée illimitée tant que I01 reste fermé. L'appareil fonctionne alors comme un indicateur de la valeur instantanée du tremblement
- Après quelques secondes, lorsque V9 atteint le seuil V12, la tension de commande V11 devient négative, ce qui bloque la diode T07 et permet à la détection du minimum de rentrer en fonction. Pendant la mesure, si la tension V7 sur l'entrée négative de l'ampli détecteur de minimum G09 est supérieure à celle du tremblement V6 arrivant sur l'entrée positive, la sortie de l'ampli détecteur de minimum G09 impose à son entrée négative de diminuer. Ceci impose à la charge de la capacité mémoire C05 d'être égale, pendant la mesure, au minimum atteint par V6 depuis le début de la mesure.
- Lorsque, à la fin de la mesure, la tension V9 devient inférieure au potentiel de masse, la tension de commande V15 devient positive et impose à travers D05 une tension positive au point commun T09-R29, ce qui bloque la diode de détection du minimum T09. Il en résulte que V7 et V8 ne peuvent plus diminuer, ce qui revient à verrouiller le contenu de la capacité mémoire C05. Simultanément le transistor inverseur T10 commandé par la tension de commande V15, devient conducteur et la tension affichée V14 est égale à V8, c'est à dire le minimum absolu du tremblement pendant la mesure, résultat qui est contenu dans la capacité-mémoire C05. Simultanément, la fin de la mesure est signalée à l'utilisateur par le moyen de visualisation DEL01 asservi lui aussi à la temporisation C01-R07. En effet, lorsque la tension de commande V15 devient positive, la diode D12 devient conductrice, ce qui bloque l'oscillateur du bloc i (figure 6G).
- encore plus tard, après que résultat de la mesure ait été affiché pendant un certain temps, lorsque la capacité de temporisation C01 est suffisamment déchargée, le transistor de mise hors-tension T06 cesse d'être conducteur et la borne (-) du dispositif n'est plus alimentée. L'appareil s'éteint alors automatiquement.

Lorsque le commutateur I01 est fermé, la capacité de temporisation C01 reste chargée au maximum, l'appareil reste donc sous tension en permanence et affiche la valeur instantanée du tremblement.

Le bloc i présenté sur la figure 6G est un oscillateur qui commande la diode de signalisation DEL01. L'oscillateur est constitué d'un amplificateur opérationnel G12 commandé par la tension de commande V15 à travers la diode D12. Pendant la mesure, la tension de commande V15 est négative et l'utilisateur sait que la mesure est en cours par le clignotement de la diode de signalisation DEL01. Lorsque la tension de commande V15 devient positive à la fin de la mesure, la diode D12 devient conductrice, ce qui bloque l'oscillateur. Le clignotement cesse et l'utilisateur est ainsi averti de la fin de la mesure.

La figure 7 montre un dispositif qui intègre le capteur (1), le circuit (2) et l'affichage (3) dans un même boitier (4). Ce mode de réalisation présente l'avantage d'un moindre encombrement et d'une plus grande facilité d'emploi. Le capteur pourra alors, par exemple, être un capteur d'accélérations situé à l'intérieur du boitier de l'appareil, l'ensemble de l'appareil étant miniaturisé et fonctionnant sur piles peut notamment être tenu dans la main ou rendu solidaire, d'une autre façon, de l'organisme dont on souhaite mesurer les vibrations (par exemple fixation à l'aide d'une ceinture autour du bras ou de la taille, ou de la tête...). Cette forme de réalisation s'applique particulièrement à la mesure du stress chez l'homme.

La figure 8 montre la courbe de réponse en amplitude du capteur présenté sur la figure 4. Cette courbe est quasi-linéaire pour les fréquences de l'ordre de 10 Hz, ce qui fait qu'un tel capteur est un excellent capteur pour la mesure du tremblement humain.

On a vu que la mesure du tremblement entraine la nécessité d'une mémoire C05 de stockage du tremblement minimum atteint depuis le début de la mesure. Une façon de permettre la mesure de vibrations pendant un temps prédéterminé T consiste, le résultat de la mesure étant conservé dans la mémoire C05, à lancer la mesure par un organe de déclenchement, par exemple le bouton-poussoir PS01, dont le déclenchement entraine :
- la remise à zéro d'une temporisation C01-R07 déterminant la durée de la mesure, cette durée étant éventuellement réglable.
- l'initialisation de la mémoire C05 qui contiendra le résultat de la mesure.

Dans le cas où l'on mesure le minimum absolu des vibrations pendant la durée de la mesure, C05 sera initialisé par l'organe de déclenchement à une valeur supérieure au résultat de la mesure attendue.

On sait que la puissance dissipée par un objet qui vibre est proportionnelle au carré de l'amplitude. L'élévation ou carré du signal par le bloc 3 des figures 5 et 6C permet donc d'obtenir en utilisant un capteur d'accélérations un signal affiché proportionnel à la puissance dissipée sous forme de vibrations. L'élévation au carré présente aussi l'avantage d'augmenter la dispersion des résultats de mesure.

La durée de la mesure doit être choisie de telle manière que l'objet de la mesure atteigne, au moins une fois pendant la durée de la mesure, son tremblement de base. Les expériences ont montré qu'une durée comprise entre 5 secondes et 5 minutes convient parfaitement, la durée optimale pour les mesures chez l'homme étant de l'ordre de vingt secondes, et pour les mesures chez la souris de l'ordre de 5 minutes.

Le signal tel qu'il parvient en V5 a été amplifié, corrigé, redressé et élevé au carré. Cependant, dans le cas par exemple d'un signal d'entrée sinusoïdal de période 15 Hz, le signal en V5 passe par zéro tous les 1/30 ièmes de seconde. Une détection directe du minimum absolu pendant un temps de t'ordre de la minute donnerait donc toujours zéro s'il n'y avait pas de lissage (bloc 4 de la figure 6). La constante de temps du lissage est éventuellement réglable par les valeurs de C06 et R03. Ceci trouve application en particulier pour les usages de laboratoire dans lesquels on souhaite traiter le signal en faisant varier la constante de temps du lissage. A la fin de la mesure, le résultat reste affiché pendant un temps suffisant pour permettre sa lecture, par exemple jusqu'à la mesure suivante ou jusqu'à l'extinction de l'appareil. Dans le dispositif présenté sur la figure 6, la durée de la mesure est de l'ordre d'une vingtaine de secondes et le résultat reste affiché une vingtaine de secondes à la fin de la mesure, après quoi l'appareil s'éteint automatiquent.

Dans le dispositif de la figure 6, la diode DEL01 est clignotante pendant la mesure, éteinte lorsque l'appareil est hors-tension et allumée lors de l'affichage du résultat, ce qui permet à l'utilisateur de distinguer facilement les trois états possibles de l'appareil : mesure en cours, mesure terminée ou appareil hors-tension. De plus une diode clignotante pendant la mesure permet de capter l'attention du sujet, d'où une utilisation plus agréable, une plus grande concentration du sujet, et une diminution des éventuels mouvements parasites d'origine musculaire.

La durée T de la mesure est réglable par exemple en remplaçant R07 par un potentiomètre. Ceci peut trouver application par exemple pour un appareil utilisé en laboratoire, la durée de la mesure étant variable suivant les animaux utilisés, la nature des mesures à effectuer...

Le potentiomètre P01 permet un étalonnage de l'appareil en fin de chaine de fabrication, ou peut permettre de faire des mesures avec un gain variable, ce qui trouve une application en particulier pour un usage en laboratoire où l'on voudrait faire des mesures de nature différente sur le même appareil : par exemple parce que l'on souhaite utiliser des capteurs différents sur le même appareil ou tenir compte d'un facteur de correction ou si l'on souhaite étalonner l'appareil avant une série de mesures.

Le dispositif peut afficher pendant la mesure soit la valeur instantanée du tremblement, soit le contenu de la mémoire C05, soit d'autres chiffres. L'affichage de la valeur instantanée du tremblement pendant la mesure présente l'avantage pour l'utilisateur de pouvoir suivre l'évolution de son tremblement en fonction de ses mouvements, de son état psychique, de la profondeur de son état de méditation... Le dispositif peut alors être utilisé en biofeedback, c'est à dire que l'utilisateur apprend à ne plus trembler car il voit instantanément son tremblement augmenter ou diminuer en fonction de ses réactions. Le dispositif de l'invention possède donc des applications au niveau de la rééducation des individus qui tremblent (vieillards, parkinsoniens, anciens alcooliques...).

Le dispositif de la figure 6 peut aussi être utilisé comme un indicateur de tremblement pendant une durée plus longue que la durée déterminée d'une mesure, par exemple par fermeture de l'interrupteur I01. Un tel dispositif possède ainsi deux fonctions : indicateur du minimum de tremblement pendant une durée prédéterminée ou indicateur du tremblement instantané pendant une durée indéterminée.

Les dispositifs pour la mise en oeuvre de l'invention peuvent être miniaturisés par l'utilisation d'un microprocesseur. Il est même possible d'envisager la fabrication de montres qui indiquent en plus de l'heure le degré de tremblement (ou taux de stress) grâce à l'utilisation d'un capteur d'accélérations miniaturisé. Des capteurs miniatures de ce type sont déjà utilisés à l'heure actuelle, en particulier pour certaines applications militaires. L'utilisation d'un microprocesseur permet sans difficulté de réduire la dimension du circuit et de l'affichage de manière à tenir dans une montre.

Il est aussi possible de réaliser un dispositif mixte qui mesurerait plusieurs paramètres physiologiques : par exemple le rythme cardiaque, la tension artérielle et le tremblement nerveux. Un boitier et un affichage communs aux trois fonctions peuvent être reliés à un brassard équipé d'un accéléromètre qui mesure le tremblement nerveux du bras et d'un capteur de pression qui mesure la tension artérielle et le rythme cardiaque.

L'invention n'est bien entendu pas limitée aux détails des exemples de réalisation qui sont décrits ci-dessus à titre de simple illustration. Sans sortir du cadre de l'invention, on peut envisager d'autres dispositifs et d'autres moyens de mise en oeuvre que ceux présentés, en particulier par l'utilisation d'un circuit électronique comportant un ou plusieurs micro-processeurs.

L'invention est particulièrement destinée aux applications suivantes (liste non limitative):
1) appareil grand public permettant la mesure du tremblement, du stress et du degré de nervosité chez l'homme, par exemple au moyen d'un capteur d'accélérations tenu dans la main ou intégré dans le boitier de l'appareil, celui-ci étant alors miniaturisé pour tenir tout entier dans la main.
2) appareil grand public ou médical permettant de suivre l'évolution du tremblement microscopique à l'occasion du sevrage du tabac, du sevrage du café, d'un changement de régime alimentaire vers une alimentation plus naturelle (aliments crus), d'une cure thermale, d'une séance de relaxation...
3) appareil médical permettant de suivre l'évolution du tremblement chez les malades atteints de tremblements pathologiques (Parkinson, tremblements séniles, sevrage alcoolique, nervosité...) ou chez les personnes traitées par des médicaments touchant le système nerveux (somnifères, barbituriques, calmants, antidépresseurs...).
4) appareil de laboratoire permettant de mesurer le tremblement microscopique de l'homme, de la souris ou d'autres organismes vivants après injection ou ingestion de diverses substances (expérimentations en pharmacologie, effet de différents aliments...).
5) appareil de laboratoire permettant la détection d'aliments irradiés (il n'existe actuellement aucun moyen de mesure permettant de déterminer après coup si un aliment a été ou non irradié). Le principe est le suivant : les animaux nourris de façon 100% naturelle (aliments naturels et crus) sont très sensibles aux dénaturations subies par les aliments. L'ingestion de substances artificielles ou d'aliments irradiés induit un tremblement pathologique chez ces animaux qui est invisible à l'oeil nu mais mesurable. On pourra utiliser par exemple des souris. Toute dénaturation selon un procédé qui n'existe pas dans la nature et qui entraine une modification même minime de la structure moléculaire de l'aliment (ce qui est le cas de l'irradiation), entraine une variation du tremblement microscopique de l'animal dans les heures ou les jours qui suivent l'absorption. On peut donc procéder par exemple de la façon suivante: séparer un lot de souris dont l'alimentation est 100% naturelle depuis un temps suffisant en 2 groupes identiques A et B. Le groupe A est nourri avec l'aliment à tester (par exemple des pommes de terre dont on ignore si elles ont été irradiées ou non) et le groupe témoin B est nourri avec le même aliment non-irradié ni traité (pommes de terre biologiques dans cet exemple). Si les pommes de terre ont été irradiées, on observera une modification du tremblement microscopique dans les souris du groupe A par rapport à celles du groupe B. A défaut d'un aliment témoin absolument identique et 100% naturel pour le groupe B, on pourra utiliser un autre aliment, à condition qu'il soit lui aussi 100% naturel. Les animaux pourront ne manger que cet aliment pendant plusieurs jours, durée pendant laquelle sera mesuré le tremblement microscopique.
   D'une façon plus générale, on remarquera que le dispositif selon l'invention permet avec un protocole de ce type de détecter la présence de substances neurologiquement actives dans les aliments (viande d'animaux traités par des médicaments ou ayant eux-mêmes ingéré des substances non-naturelles, aliments chauffés au delà des températures naturelles (environ 40°C), additifs alimentaires, traitements chimiques avant ou après récolte...)
6) appareil permettant le suivi du stress des conducteurs d'automobiles, pilotes d'avion, pilotes de courses automobiles...
7) appareil de mesure des réactions en situation de stress pour l'entrainement et/ou le recrutement d'astronautes, pilotes d'avions, dirigeants d'entreprises, pilotes d'engins ou tout autre situation requérant une bonne maîtrise du stress...
8) appareil permettant la mise au point et le contrôle de l'efficacité de méthodes anti-stress (relaxation, méditation, thalassothérapie, gymnastique....)
9) application au suivi du degré de nervosité chez les prisonniers, permettant par exemple de ne pas relâcher un prisonnier trop nerveux.
10) application de la mesure du tremblerment nerveux à la détection de mensonges lors d'enquêtes judiciaires.
11) appareil grand public de mesure du degré de nervosité (stress) miniaturisé, par exemple sous la forme d'une montre qui indique d'une part l'heure, et d'autre part le degré de nervosité qui peut être totalisé au cours de la journée, ou pendant un certain temps.
12) application de l'invention à la mesure de vibrations d'objets autres que des organismes vivants, par exemple habitations, carosseries d'automobiles, fuselages d'avions, trains, fusées, mesures sismiques...

## Revendications

1. Procédé de mesure de vibrations, notamment du tremblement nerveux des organismes vivants, la mesure comportant les opérations suivantes : transformation des vibrations en signal électrique --- traitement du signal --- affichage ou transmission du résultat vers d'autres appareils, caractérisé par le fait que le traitement du signal consiste à mesurer l'amplitude du signal en distinguant les valeurs les plus basses de l'amplitude des valeurs plus hautes de manière à ne prendre en compte que les valeurs les plus basses, c'est-à-dire du tremblement au repos, tout en éliminant les plus hautes.

2. Procédé de mesure de vibrations selon la revendication 1 caractérisé par le fait que le traitement du signal comporte la détection d'un minimum.

3. Procédé de mesure de vibrations selon la revendication 1 ou 2 caractérisé en ce que le traitement du signal comporte une amplification, un redressement, une élévation au carré, un lissage et la mesure du minimum absolu de l'amplitude du signal pendant la durée de la mesure.

4. Procédé de mesure de vibrations selon l'une quelconque des revendications précédentes caractérisé en ce que l'objet de la mesure est un homme qui tient le capteur piézo-électrique dans sa main, le dit capteur étant par exemple d'une forme non-symétrique ne peut être tenu que d'une seule manière, de façon à imposer au sujet l'orientation du capteur.

5. Procédé de mesure des vibrations conforme à l'une quelconque des revendications précédentes caractérisé par le fait que :
- le résultat de la mesure reste affiché à l'issue de la mesure pendant un certain temps, par exemple jusqu'à la mesure suivante ou jusqu'à ce que l'appareil soit mis hors tension.
- l'utilisateur est averti de la fin de la mesure par un moyen de visualisation, par exemple par l'allumage ou le clignotement d'une diode électro-luminescente (DEL01) ou par un clignotement de l'affichage.

6. Procédé selon l'une des revendications précédentes caractérisé par le fait que la mesure est lancée par un organe de déclenchement, en particulier par un bouton-poussoir, dont le déclenchement entraine :
- la remise à zéro d'une temporisation (C01-R07) déterminant la durée de la mesure, cette durée étant éventuellement réglable.
- l'initialisation d'une mémoire d'enregistrement du résultat (C05) à une valeur supérieure au résultat de la mesure attendue.

7. Procédé selon l'une quelconque des revendications précédentes pour la détection d'aliments altérés, et en particulier d'aliments irradiés, caractérisé en ce que l'on mesure le tremblement de plusieurs lots d'animaux, l'un des lots étant nourri avec l'aliment à tester et un lot témoin recevant une alimentation de référence pouvant notamment être exclusivement constituée d'aliments 100% naturels.

8. Dispositif pour la mise en oeuvre du procédé selon l'une des revendications 1 à 7 qui comprend, en coopération avec un capteur piézo-électrique :
- une temporisation constituée par un condensateur (C01) monté en parallèle avec une résistance (R07).
- un organe de déclenchement (PS01) qui initialise la temporisation par charge du condensateur (C01).
- un moyen (C05) de mémorisation du résultat de la mesure également asservi au dit-moyen de déclenchement (PS01).

9. Dispositif pour la mise en oeuvre du procédé selon l'une des revendications 1 à 7 caractérisé en ce qu'il comporte un commutateur (I01) qui permet de mesurer au choix soit la valeur instantanée du tremblement, soit le signal élaboré selon la revendication 1.

10. Dispositif pour la mise en oeuvre du procédé de détection du minimum conforme à la revendication 3 caractérisé par un filtre passe-bas (R03-C06) suivi d'un amplificateur opérationnel (G09) alimentant la capacité-mémoire (C05) par l'intermédiaire d'une diode (T09).

11. Dispositif selon la revendication 8 caractérisé en ce que la temporisation (C01-R07) est associée à un ensemble de moyens de séquencement comprenant:
- un moyen (G15) d'initialisation de la capacité-mémoire (C05) par l'intermédiaire d'une diode (T07).
- un moyen (G11-D05) de verrouillage de la charge de la capacité mémoire (C05).
- un moyen de mise hors-tension (T06).

12. Capteur d'accélérations pour la mise en oeuvre du procédé selon l'une des revendications 1 à 7 caractérisé en ce que la transformation des vibrations en signal électrique est effectuée par un élément piézo-électrique (101) relié à un équipage mobile en flexion (103), dont les mouvements sont induits par l'inertie d'une masselotte (104) et dont la course est limitée par butée (108).

13. Dispositif pour la mise en oeuvre du procédé selon l'une des revendications 1 à 7 caractérisé par le fait que le capteur est constitué d'un élément piézo-électrique (1) rendu solidaire d'une lame (33) reliée d'une part à un socle fixe (32), et d'autre part par l'intermédiaire d'un organe de liaison (22) à l'objet de la mesure (23), la course de la lame étant limitée par butée (34) et la position de la jonction entre l'organe de liaison (22) et la lame (33) étant réglable par déplacement du point de jonction (35).

## Patentansprüche

1. Verfahren zum Messen von Vibrationen, insbesondere des nervösen Tremors lebender Organismen, wobei die Messung folgende Operationen beinhaltet: Umsetzung der Vibrationen in ein elektrisches Signal - Behandeln des Signals - Anzeigen oder Übertragen des Ergebnisses zu anderen Vorrichtungen, dadurch **gekennzeichnet,** daß die Behandlung des Signals darin besteht, die Signalamplitude zu messen, um die niedrigsten Amplitudenwerte von den höchsten zu unterscheiden, derart, daß bei Eliminierung der höchsten Werte lediglich die niedrigsten Werte, d.h. des Ruhe-Tremors, berücksichtigt werden.

2. Verfahren zum Messen von Vibrationen nach Anspruch 1, dadurch **gekennzeichnet**, daß die Signalbehandlung das Erfassen eines Minimums umfaßt.

3. Verfahren zum Messen von Vibrationen nach Anspruch 1 oder 2, dadurch **gekennzeichnet,** daß die Signalbehandlung eine Verstärkung, eine Gleichrichtung, eine Quadrierung, eine Glättung und eine Messung des Absolut-Minimums der Amplitude des Signals während des Verlaufs der Messung beinhaltet.

4. Verfahren zum Messen von Vibrationen nach irgendeinem der vorhergehenden Ansprüche, dadurch **gekennzeichnet,** daß das Meßobjekt ein Mensch ist, der in seiner Hand einen piezoelektrischen Aufnehmer hält, der, indem er beispielsweise eine unsymmetrische Form aufweist, nur in einer bestimmten Weise gehalten werden kann, so daß der Person die Orientierung des Aufnehmers aufgezwungen wird.

5. Verfahren zum Messen von Vibrationen nach irgendeinem der vorhergehenden Ansprüche, dadurch **gekennzeichnet,** daß
- das Meßergebnis auf der Meßwertausgabe über eine gewisse Zeitspanne angezeigt bleibt, beispielsweise bis zur nachfolgenden Messung oder bis das Gerät spannungslos gemacht ist,
- der Benutzer durch visuelle Mittel über das Meßende informiert wird, beispielsweise durch Leuchten oder Blinken einer Elektroluminenszensdiode (DEL01) oder durch ein Blinken der Anzeige.

6. Verfahren nach einem der vorhergehenden Ansprüche, dadurch **gekennzeichnet,** daß die Messung durch ein Auslöseorgan eingeleitet wird, insbesondere durch einen Druckknopf, dessen Auslösung zur Folge hat:
- die Nullsetzung eines die Meßdauer bestimmenden Zeitglieds (C01-R07), wobei diese Dauer möglicherweise regulierbar ist,
- die Initialisierung eines Ergebnisspeichers (C05) mit einem Wert, der oberhalb des erwarteten Meßergebnisses liegt.

7. Verfahren nach irgendeinem der vorhergehenden Ansprüche für die Untersuchung von behandelten Nahrungsmitteln, insbesondere bestrahlten Nahrungsmitteln, dadurch **gekennzeichnet,** daß man den Tremor mehrerer Gruppen von Lebewesen mißt, von denen eine Gruppe mit dem zu prüfenden Nahrungsmittel gespeist worden ist, und eine Vergleichsgruppe ein Bezugs-Nahrungsmittel erhält, welches insbesondere ausschließlich aus zu 100% natürlichen Nahrungsmitteln bestehen kann.

8. Vorrichtung zum Durchführen des Verfahrens nach einem der Ansprüche 1 bis 7, die in Verbindung mit einem piezoelektrischen Aufnehmer aufweist:
- ein Zeitglied, gebildet durch einen Kondensator (C01), der parallel zu einem Widerstand (R07) geschaltet ist,
- ein Auslöseorgan (PS01), welches das Zeitglied durch Aufladen des Kondensators (C01) initialisiert,
- ein Mittel (C05) zum Speichern des Meßergebnisses, welches ebenfalls von dem Auslösemittel (PS01) gesteuert wird.

9. Vorrichtung zur Durchführung des Verfahrens nach einem der Ansprüche 1 bis 7, dadurch **gekennzeichnet,** daß sie einen Umschalter (I01) aufweist, der das wahlweise Messen des Augenblickswerts des Tremors oder des gemäß Anspruch 1 verarbeiteten Signals gestattet.

10. Vorrichtung zum Durchführen des Verfahrens des Erfassens des Minimumwerts gemäß des Anspruch 3, **gekennzeichnet** durch ein Tiefpassfilter (R03-C06), an das ein Operationsverstärker (G09) angeschlossen ist, der über eine Diode (T09) den Kapazitäts-Speicher (C05) speist.

11. Vorrichtung nach Anspruch 8, dadurch **gekennzeichnet,** daß das Zeitglied (C01-R07) zu einer Ablaufsteuerung gehört, welche aufweist:
- ein Mittel (G15) zum Initialisieren des Kapazitäts-Speichers (C05) über eine Diode (T07),
- ein Mittel (G11-D05) zum Blockieren der Ladung des Kapazitäts-Speichers (C05),
- ein Mittel zur Spannungs-Abschaltung (T06).

12. Beschleunigungsaufnehmer zur Ausführung des Verfahrens nach einem der Ansprüche 1 bis 7, dadurch **gekennzeichnet,** daß die Umsetzung der Vibrationen in ein elektrisches Signal durch ein piezoelektrisches Element (101) erfolgt, welches mit einer beweglichen Biegeanordnung (103) verbunden ist, deren Bewegungen durch die Trägheit eines Regulierungsgewichts (104) verursacht und deren Bewegungshub durch einen Anschlag (108) begrenzt ist.

13. Vorrichtung zum Durchführen des Verfahrens nach einem der Ansprüche 1 bis 7, dadurch **gekennzeichnet**, daß der Aufnehmer gebildet wird durch ein piezoelektrisches Element (1), welches einstückig mit einem Blättchen (33) ist, welches einerseits mit einem feststehenden Sockel (32) verbunden ist, und andererseits über ein Verbindungsorgan (22) mit einem Meßobjekt (23) verbunden ist, wobei der Bewegungshub des Blättchens durch einen Anschlag (34) begrenzt ist und die Lage der Verbindung zwischen dem Verbindungsorgan (22) und dem Blättchen (33) durch Versetzung des Verbindungspunkts (35) regulierbar ist.

## Claims

1. Process for measuring vibrations, in particular nervous trembling in living organisms, the measurement including the following operations : conversion of the vibrations into an electrical signal - signal processing - display or transmission of the result to other equipment, characterised by the fact that the signal processing consists of measuring the amplitude of the signal and distinguishing the lowest values of the amplitude from higher values in order to take into account only the lowest values, i.e. of trembling when at rest, and eliminate the higher values.

2. Vibration measurement process according to claim 1, characterised by the fact that the signal processing includes detection of a minimum value.

3. Vibration measurement process according to claim 1 or claim 2, characterised in that the signal processing comprises amplifying, rectifying, squaring, smoothing and measuring the absolute minimum amplitude of the signal during the measurement period.

4. Vibration measurement process according to any one of the preceding claims, characterised in that the measurement subject is a person who holds the piezoelectric sensor in his hand, the said sensor having a non-symmetrical shape, for example, so that it can be held in only one way, so that the subject is required to orient the sensor correctly.

5. Vibration measurement process according to any one of the preceding claims, characterised by the fact that :
- the measurement result continues to be displayed on completion of the measurement for a certain time, for example until the next measurement or until the equipment is switched off,
- the user is advised of the end of the measurement by display means, for example by a light-emitting diode (DEL01) that is turned on or blinks on and off or by the display blinking on and off.

6. Process according to any one of the preceding claims, characterised by the fact that the measurement is started by a trigger unit, in particular by a push-button, operation of which :
- resets a time-delay (C01-R07) determining the measurement duration, which may be variable,
- initializes a memory for storing the result (C05) at a value higher than the expected measurement result.

7. Process according to any one of the preceding claims for detecting foodstuffs that have been adulterated, in particular irradiated foodstuffs, characterised in that the trembling of several batches of animals is measured, one of the batches being fed with the foodstuff under test and a control batch receiving reference foodstuffs that may comprise 100% natural foodstuffs only.

8. Device for implementing the process according to any one of claims 1 to 7, comprising, in conjunction with a piezo-electric sensor :
- a time-delay in the form of a capacitor (C01) connected in parallel with a resistor (R07),
- a trigger unit (PS01) which initializes the time-delay by charging the capacitor (C01),
- means (C05) for storing the measurement result also conditioned by said trigger means (PS01).

9. Device for implementing the process according to any one of claims 1 to 7, characterised in that it comprises a switch (I01) selecting measurement either of the instantaneous trembling value or of the signal processed according to claim 1.

10. Device for implementing the minimum value detection process according to claim 3, characterised by a low-pass filter (R03-C06) followed by an operational amplifier (G09) feeding the memory capacity (C05) through a diode (T09).

11. Device according to claim 8, characterised in that the time-delay (C01-R07) is associated with a set of sequencing means comprising :
- means (G15) for initializing the memory capacity (C05) through a diode (T07),
- means (G11-D05) for latching the charge in the memory capacity (C05),
- switch-off means (T06).

12. Acceleration sensor for implementing the process according to any of claims 1 to 7, characterised in that the vibrations are converted into electrical signals by a piezo-electric element (101) connected to an assembly (103) which is mobile in flexion, the movements of which are induced by the inertia of a counterweight (104) and the travel of which is limited by abutment means (108).

13. Device for implementing the process according to any one of claims 1 to 7, characterised by the fact that the sensor comprises a piezo-electric element (1) attached to a blade (33) joined to a fixed base (32) and through a coupling member (22) to the subject of the measurement (23), the travel of the blade being limited by abutment means (34) and the position of the junction between coupling member (22) and the blade (33) being adjustable through movement of the junction position (35).
